# EUROPEAN PATENT APPLICATION

(11) **EP 4 357 456 A1**
(43) Date of publication of application: **24.04.2024**
(21) Application number: 23204596.3
(22) Date of filing: 19.10.2023
(51) Int. Cl.: C12N 15/11, A61K 48/00, B01D 15/00, C12N 15/85, C12Q 1/00

(54) **TREATMENT OF LIVER CANCER OR LIVER FIBROSIS**

(30) Priority: 20.10.2022 US 202218048267
(71) Applicant: Twister Biotech, Inc., Houston TX 77225 (US); Baylor College Of Medicine, Houston, TX 77030 (US)
(72) Inventor: ZECHIEDRICH, E. Lynn, Houston, 77030 (US); AREVALO-SOLIZ, Lirio Milenka, Houston, 77030 (US); CATANESE Jr., Daniel James, Houston, 77225 (US); FOGG, Jonathan Marcus, Houston, 77030 (US); COKER, Christopher E., Houston, 77225 (US); AGARWAL, Sandeep, Houston, 77030 (US)
(74) Representative: Barker Brettell LLP

(57) **Abstract**

Compositions containing MiniVectors and gene therapy uses, including long term repeated gene therapy uses, to treat liver fibrosis or liver cancer.

## Description

### FEDERALLY SPONSORED RESEARCH STATEMENT

Not applicable.

### REFERENCE TO ELECTRONIC SEQUENCE LISTING

The application contains a Sequence Listing which has been submitted electronically in .XML, format and is hereby incorporated by reference in its entirety. Said .XML copy, created on December 1, 2022, is names "TWISTER-004US02.xml" and is 88,732 bytes in size. The sequence listing contained in this .XML file is part of the specification and is hereby incorporated by reference in its entirety.

### FIELD OF THE DISCLOSURE

The disclosure generally relates to methods and materials for treating liver cancer or liver fibrosis. More particularly, it relates to methods of making DNA MiniVectors and compositions comprising MiniVectors that comprises liver modulating sequences.

### BACKGROUND OF THE DISCLOSURE

Cirrhosis, also known as liver cirrhosis or hepatic cirrhosis, and end-stage liver disease, is the impaired liver function caused by the formation of scar tissue known as "fibrosis" due to damage caused by liver disease. Damage causes tissue repair and subsequent formation of scar tissue, which over time can replace normal functioning tissue leading to the impaired liver function of cirrhosis and ultimately lead to cancer and/or death.

The disease typically develops slowly over months or years. Early symptoms may include tiredness, weakness, loss of appetite, unexplained weight loss, nausea and vomiting, and discomfort in the right upper quadrant of the abdomen. As the disease worsens, symptoms may include itchiness, swelling in the lower legs, fluid build-up in the abdomen, jaundice, bruising easily, and the development of spider-like blood vessels in the skin. The fluid build-up in the abdomen may become spontaneously infected. More serious complications include hepatic encephalopathy, bleeding from dilated veins in the esophagus, stomach, or intestines, and liver cancer.

Diagnosis is based on blood tests, medical imaging, and liver biopsy. Cirrhosis is most commonly caused by alcoholic liver disease, non-alcoholic steatohepatitis (NASH) - (the progressive form of non-alcoholic fatty liver disease), chronic hepatitis B, and chronic hepatitis C. Heavy drinking over a number of years can cause alcoholic liver disease. NASH has many causes, including obesity, high blood pressure, abnormal levels of cholesterol, type 2 diabetes, and metabolic syndrome. Less common causes of cirrhosis include autoimmune hepatitis, primary biliary cholangitis, and primary sclerosing cholangitis that disrupts bile duct function, genetic disorders such as Wilson's disease and hereditary hemochromatosis, and chronic heart failure with liver congestion.

No specific treatment for cirrhosis is known, but many of the underlying causes may be treated by a number of medications that may slow or prevent worsening of the condition. Avoiding alcohol is recommended in all cases. Hepatitis B and C may be treatable with antiviral medications. Autoimmune hepatitis may be treated with steroid medications. Ursodiol may be useful if the disease is due to blockage of the bile duct. Other medications may be useful for complications such as abdominal or leg swelling, hepatic encephalopathy, and dilated esophageal veins. If cirrhosis leads to liver failure, a liver transplant may be one treatment option.

However, cirrhosis often proceeds to liver cancer. Hepatocellular carcinoma (HCC) is the most common type of primary liver cancer in adults and is currently the most common cause of death in people with cirrhosis. As with any cancer, the treatment and prognosis of HCC vary depending on the specifics of tumor histology, size, how far the cancer has spread, and overall health.

Treatment of hepatocellular carcinoma varies by the stage of disease, a person's likelihood to tolerate surgery, and availability of liver transplant, as follows:

**Curative intention:** for limited disease, when the cancer is limited to one or more areas of within the liver, surgically removing the malignant cells may be curative. This may be accomplished by resection of the affected portion of the liver (partial hepatectomy) or in some cases by orthotopic liver transplantation of the entire organ.

**"Bridging" intention:** for limited disease which qualifies for potential liver transplantation, the person may undergo targeted treatment of some or all of the known tumor while waiting for a donor organ to become available.

**"Downstaging" intention:** for moderately advanced disease that has not spread beyond the liver, but is too advanced to qualify for curative treatment. The person may be treated by targeted therapies in order to reduce the size or number of active tumors, with the goal of once again qualifying for liver transplant after this treatment.

**Palliative intention:** for more advanced disease, including spread of cancer beyond the liver or in persons who may not tolerate surgery, treatment intended to decrease symptoms of disease and maximize duration of survival.

Loco-regional therapy (also referred to as liver-directed therapy) refers to any one of several minimally-invasive treatment techniques to focally target HCC within the liver. These procedures are alternatives to surgery, and may be considered in combination with other strategies, such as a later liver transplantation. Generally, these treatment procedures are performed by interventional radiologists or surgeons, in coordination with a medical oncologist. Loco-regional therapy may refer to either percutaneous therapies (e.g. cryoablation), or arterial catheter-based therapies (chemoembolization or radioembolization).

Although we have treatment options, each year in the United States, about 24,500 men and 10,000 women get liver cancer, and about 18,600 men and 9,000 women die from the disease. Thus, what is needed in the art are better methods of treating the fibrosis and cancer resulting from liver disease. The ideal method would delay or even reverse the progression of the disease.

This invention addresses one or more of those needs.

### SUMMARY OF THE DISCLOSURE

This application focuses on the preparation and usage of MiniVectors having one or more of the following payloads used to treat liver fibrosis and/or cancer. The payload or "liver modulating sequence" ("LMS") may function to: Upregulate P53 (UniProt P04637); Upregulate Relaxin (aka REL2 or rln2 at UniProt P04090); Knock down FOXM1 (Forkhead box protein M1 at UniProt Q08050); Knock down CAD11(cadherin 11 or CDH11 at UniProt P55287); Knock down MDM2 (E3 ubiquitin-protein ligase Mdm2 at UniProt Q00987); Knock down MDM4 (at UniProt 015151); Knock down STATS (Signal transducer and activator of transcription 3 at UniProt P40763); Knock down STAT6 (UniProt P42226); Knock down TGFB1 (UniProt P01137) or combinations thereof.

In addition, the MiniVector cassettes could encode for one or more payloads on a single MiniVector. Alternatively, the treatment could comprise different MiniVectors for the different payloads.

The MiniVectors could be delivered in an array of excipients (e.g., lipid nanoparticles, polymer nanoparticles, exosomes, silicon nanoparticles, mesoporous silica nanoparticles). They may also employ excipients with one or more targeting moieties or conjugate groups (e.g., aptamers, GalNac) to improve therapy specificity.

The MiniVectors plus excipients could be delivered via IV, aerosol, direct injection, surgical methods, or other pharmaceutically acceptable routes of administration.

Ideally, the MiniVector will have less than 0.1%, or even less than 0.02% contamination by parent plasmid sequences, including unrecombined parent plasmid and the "miniplasmid" byproduct which contains the unwanted part of the plasmid that is not incorporated into the MiniVector.

The extremely high purity, optionally combined with CpG minimization and/or human condon usage allows repeated usage over a period of months, something that is usually not possible in other gene therapies.

**FIG.** 1 shows a general schematic for making MiniVectors.

**FIG. 2** schematizes the modularity of MiniVectors. On the left is shown the simplest embodiment of a MiniVector consisting of (A) the hybrid DNA recombination sequences, *att*L or *att*R*,* that are products of the site-specific recombination, (B) a mammalian promoter, (C) the therapeutic DNA sequence (aka payload) to be expressed, and (D) a transcriptional terminator.

The MiniVector contains, for example, DNA encoding merely the transgene expression cassette (including promoter and a sequence of interest, wherein the payload sequence may be, for example, a gene, or a segment of a gene, a sequence encoding an interfering RNA (*e.g*., shRNA, IhRNA, miRNA, shRNA-embedded miRNA, IncRNA, piRNA), or a template for *e.g*., homology-directed repair, alteration, or replacement of the targeted DNA sequence). Importantly, the MiniVector is almost completely devoid of bacterial-originated sequences.

MiniVectors are also preferably designed to contain limited or no homology to the human genome. They are also typically much shorter in length than plasmids. Therefore, the frequency of integration is at least as low as the 5 × 10⁻⁶ rate of plasmid integration and likely much lower. Designed to be preferably delivered locally, any non-target would have to have MiniVector in the non-target cells/tissue to cause an off-target effect. In that way, then, MiniVectors should not have off-target effects. By contrast, many viruses are designed to integrate into the genome, and therefore there is a major risk of off-target integration with viral vectors.

As used herein, a "MiniVector" is a double-stranded, supercoiled circular DNA typically lacking a bacterial origin of replication or an antibiotic selection gene, and having a length of about 250 bp - 500 bp (exclusive of payload), and having much higher purity than minicircles.

As used herein, "recombinant side products" include the miniplasmid that contains the unwanted bacterial sequences (origin of replication and antibiotic selection gene). The miniplasmid is considered the "deletion product" following the intramolecular site-specific recombination and contains all the unwanted sequences of the original parent plasmid minus the MiniVector sequence. The miniplasmid is typically very large (> 3 kb) compared to the MiniVector and is removed through the purification steps of e.g., polyethylene glycol (PEG) precipitation and gel filtration.

The recombination process that generates MiniVectors also sometimes generates double-length or even triple-length MiniVectors (or higher multimers), especially for very small MiniVectors. These multimeric forms result from intermolecular site-specific recombination between sites on two separate plasmids prior to intramolecular recombination between sites on the same plasmid. These multimers do not constitute "contaminants" because they still contain **only** the therapeutic sequence, but are merely double (or triple, etc.) the desired length. Increasing the size of the MiniVectors decreases the likelihood of multimers forming (Fogg et al. 2006). Furthermore, if a homogenous preparation of single-length MiniVector is desired, an extra gel filtration step typically separates higher multimers from single unit-sized MiniVector.

As used herein, the "payload" refers to the therapeutic sequence being delivered by the MiniVector and it can be e.g., a gene or portion thereof or an inhibitory RNA. The term "expressible payload sequence" includes the payload plus any sequences needed for mammalian expression, such as promoters, terminators, enhancers and the like.

As used herein, "parent" sequences are the originating sequences from which the MiniVector was designed and made. A MiniVector will have originated from parent plasmid sequences, plus the payload itself and its various expression components such as promoters, terminators and the like, will each have a parent sequence. Each of these parent sequences can be modified to reduce CpG motifs and/or be codon optimized for use in humans.

As used herein, a "catenane" is a complex organic molecule (e.g., DNA) containing two or more macrocyclic ring structures intertwined like links of a chain.

As used herein, the term "RNA interference," or "RNAi," refers to the process whereby sequence-specific, post-transcriptional gene silencing is initiated by an RNA that is homologous in sequence to the silenced gene. RNAi, which occurs in a wide variety of living organisms and their cells, from plants to humans, has also been referred to as post-transcriptional gene silencing and co-suppression in different biological systems. The sequence-specific degradation of mRNA observed in RNAi is mediated by small (or short) interfering RNAs (siRNAs).

As used herein, the term "interfering RNA" means an RNA molecule capable of decreasing the expression of a gene having a nucleotide sequence at least a portion of which is substantially the same as that of the interfering RNA. As known in the art, interfering RNAs can be "small interfering RNAs," or siRNAs, composed of two complementary single-stranded RNAs that form an intermolecular duplex. Interfering RNAs can also be "short hairpin RNAs", or shRNAs, expressed as a single RNA strand which folds upon itself to form a hairpin. Interfering RNAs can also be "long hairpin RNAs," or IhRNAs, which are shRNA-like molecules with longer intramolecular duplexes and contain more than one siRNA sequence within the duplex region.

As used herein, the term "gene silencing" refers to a reduction in the expression product of a target gene. Silencing may be complete, in that no final gene product is detectable, or partial, in that a substantial reduction in the amount of gene product occurs.

As used herein, "shRNA" is short hairpin RNA or small hairpin RNA, and "IhRNA" is long hairpin RNA, both of which can be used to silence target gene expression via RNAi.

As used herein, "miRNA" is microRNA-a small non-coding RNA molecule (containing about 22 nucleotides) found in plants, animals, and some viruses, that functions in RNA silencing and post-transcriptional regulation of gene expression. Alternative to a contiguous duplex shRNA is an shRNA sequence embedded in a microRNA stem loop (e.g., MiRE), which may be used because it can be processed more efficiently in mammalian cells leading to more robust knockdown of the expression of the target gene. The more efficient processing of the microRNA stemloop relies on both Drosha and Dicer, whereas the contiguous duplex shRNA relies only on Dicer to cut the guide RNA that will be inserted into the RNA-induced silencing complex.

As used herein, "lncRNA" are long non-coding RNAs. These lncRNAs are a large and diverse class of transcribed RNA molecules with a length of more than 200 nucleotides that do not encode proteins (or lack >100 amino acid open reading frame). lncRNAs are thought to encompass nearly 30,000 different transcripts in humans, hence lncRNA transcripts account for a major part of the non-coding transcriptome. lncRNA discovery is still at a preliminary stage, but there are many specialized lncRNA databases, which are organized and centralized through RNAcentral (rnacentral.org). lncRNAs can be transcribed as whole or partial natural antisense transcripts to coding genes, or located between genes or within introns. Some lncRNAs originate from pseudogenes. lncRNAs may be classified into different subtypes (Antisense, Intergenic, Overlapping, Intronic, Bidirectional, and Processed) according to the position and direction of transcription in relation to other genes.

Piwi-interacting RNA or "piRNA" is the largest class of small non-coding RNA molecules expressed in animal cells. piRNAs form RNA-protein complexes through interactions with PIWI family proteins. These piRNA complexes have been linked to both epigenetic and post-transcriptional gene silencing of retrotransposons and other genetic elements in germ line cells, particularly those in spermatogenesis. They are distinct from miRNA in size (26-31 nt rather than 21-24 nt), lack of sequence conservation, and increased complexity.

The term "treating" includes both therapeutic treatment and prophylactic treatment (reducing the likelihood of disease development). The term means decrease, suppress, attenuate, diminish, arrest, or stabilize the development or progression of a disease (*e.g.,* a disease or disorder delineated herein), lessen the severity of the disease, or improve the symptoms associated with the disease.

MiniVectors can be delivered in a variety of "pharmaceutically acceptable excipients" including saline, a solvent, a branched or linear polymer (e.g., Star polymer, a liposome, a hydrogel, a lipid nanoparticle, a silicon nanoparticle, a mesoporous silica nanoparticle, a naturally occurring vesicle (e.g., an exosome), hybrids of the foregoing, or others. MiniVectors can be conjugated with a variety of ligands, agents, sugars (e.g., GalNac), nucleic acids, peptides, proteins, DNA cages, aptamers, hybrids, or other moieties to improve transfection and/or facilitate specific delivery.

As described herein, the MiniVector for use in gene therapy is present in an effective amount to treat some disease. As used herein, the term "effective amount" refers to an amount which, when administered in a proper dosing regimen, is sufficient to treat (therapeutically or prophylactically) the target disorder or symptoms of the target disorder. For example, an effective amount is sufficient to reduce or ameliorate the severity, duration, or progression of the disorder being treated, prevent the advancement of the disorder being treated, cause the regression of the disorder being treated, or enhance or improve the prophylactic or therapeutic effect(s) of another therapy.

By "reducing" or "knocking down" the expression of a target protein, we mean a reduction of at least 10%, as the body's own immune response may thereby be sufficient to target and kill cancer cells, particularly in a combination therapy combined with an immune-boosting treatment, such as CpG motifs, cytokines (chemokines, interferons, interleukins, lymphokines, and tumor necrosis factors). Preferably the reduction is at least 20%, 30% or 40%, but typically a complete knockout is not required, and indeed, can contribute to unwanted side effects.

By "upregulating" or "increasing" the expression of a target protein, we mean an increase of at least 10%. Preferably the increase is at least 20%, 25%, 30% or 40%, or even greater.

"Nanoparticles" are understood to comprise particles in any dimension that are less than 500 nanometers, more preferably less than 300 nanometers, and most preferably less than 150 nanometers. The nanoparticle can be a viral vector, a component of a viral vector (e.g., a capsid), a non-viral vector (e.g., a plasmid or RNA or MiniVector), a cell, a fullerene and its variants, a small molecule, a peptide, metal and oxides thereof, linear and branched polymers, lipid nanoparticles, hybrids, exosomes, silicon, etc.

The use of the word "a" or "an" when used in conjunction with the term "comprising" in the claims or the specification means either one or more than one, unless the context dictates otherwise. The term "about" means the stated value plus or minus the margin of error of measurement or plus or minus 25% if no method of measurement is indicated. The use of the term "or" in the claims is used to mean "and/or" unless explicitly indicated to refer to alternatives only or if the alternatives are mutually exclusive.

The terms "comprise," "have," "include," and "contain" (and their variants) are open-ended linking verbs and allow the addition of other elements when used in a claim. The phrase "consisting of" is closed, and excludes all additional elements. The phrase "consisting essentially of" excludes additional material elements but allows the inclusions of non-material elements that do not substantially change the nature of the invention, such as instructions for use, buffers, and the like. Any claim or claim element introduced with the open transition term "comprising," may also be narrowed to use the phrases "consisting essentially of" or "consisting of," and vice versa. However, the entirety of claim language is not repeated verbatim in the interest of brevity herein.

### BRIEF DESCRIPTION OF THE DRAWINGS

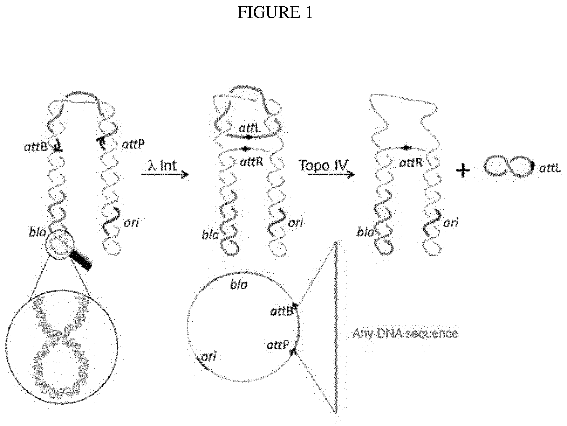

**FIG. 1****.** Generation of MiniVector DNA by λ-integrase-mediated site-specific recombination. Parent plasmid containing the sequence to be delivered flanked by *att*B and *att*P*,* the target sites for recombination. The parent plasmid is propagated in the special *E. coli* bacterial host strain, LZ54 or LZ31, harboring λ-integrase (Int) under the control of the temperature sensitive *c*I857 repressor. When the cells have reached a suitable density, expression of Int is switched on by a temperature switch. Recombination results in a catenated product containing the MiniVector. The products are decatenated, either by endonuclease cleavage of the large circle deletion product *ex vivo,* or by topoisomerase IV-mediated unlinking subsequent to the removal of topoisomerase inhibitor following the cell harvest. The deletion product containing the undesired bacterial sequences is removed, yielding pure, supercoiled MiniVector product. If desired, the MiniVector can encode *attR* and the deletion product can contain *att*L by switching the positions of *att*B and *att*P in the parent plasmid. *bla* = beta lactamase.

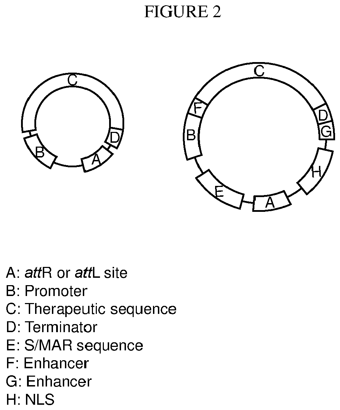

**FIG. 2****.** Modular design of MiniVectors. On the left is shown the minimal therapeutic unit, consisting only of A) *att*L or *attR* site (these sites are the products of recombination by integrase), B) a promoter, C) the therapeutic sequence (*e.g.*, **Table 1),** and D) a transcriptional terminator. The intervening regions can include any other sequence and can range in length from none to several thousand base pairs. On the right is shown a modified version containing additional modules that may be added to provide long-term persistence and expression, improve transfection, and/or facilitate nuclear localization. Any combination of these additional modules may be added to the essential modules. E) S/MAR sequence, if incorporated into the MiniVector, will be placed upstream of the transcriptional unit to utilize the dynamic negative supercoiling generated by transcription or elsewhere on the molecule. F, G) Enhancer sequences may be positioned in a number of locations, depending on the identity of the enhancer. H) Nuclear localization sequences, if incorporated, will be placed downstream of the transcriptional unit. Potential sequences for these various components are listed in **Tables 2-7.**

### DETAILED DESCRIPTION

The disclosure provides ultrapure MiniVectors that are sufficiently pure for use in gene therapy to treat liver cirrhosis or liver cancer.

### MINIVECTOR SYNTHESIS

MiniVector DNA is generated in bacterial cells using *in vivo* site-specific recombination as described previously in US7622252. In more detail, parent plasmids contain *att*B and *att*P (recognition sites for λ-integrase) oriented in the same direction. Site-specific recombination between the *att*B and *att*P sites exchanges the sequences between the two sites results in two product circles that are linked together (catenated) because of supercoiling in the parent plasmid. One of these product circles is the minivector. The other circle is the "miniplasmid", which contains the unwanted bacterial sequences. The recombination reaction also results in two new integrase sites, *att*L and *attR,* which are hybrid sites each containing sequences from *att*B and *att*P*.* If attB comes first on the parent plasmid, followed by *att*P*,* with the minivector sequence in between, the larger (~180 bp) *attR* site will end up on the minivector and the smaller (-100 bp) *att*L site is on the miniplasmid. Conversely, if *att*P comes first, followed by *att*B*,* then the *att*L site will be on the minivector. The intervening sequence between the *att*B and *att*P sites becomes incorporated into the minivector. Therefore, any sequence can be engineered into a minivector by simply cloning between the integrase sites on the parent plasmid. The system was first tested with pMC339 with *att*B preceding *att*P on the parent plasmid, which generates a 339 bp minivector containing an *attR* site and otherwise random sequence.

MiniVectors are generated using engineered *Escherichia coli* strains (examples include but are not limited to LZ31 and LZ54. These strains express, λ-integrase (λ-Int) under the tight control of the temperature-sensitive cI857 repressor. The *Escherichia coli* strain is transformed with the relevant parent plasmid. When cells are grown at 30° C., no λ-Int is expressed because of the tight control afforded by the cI857 repressor. This prevents premature recombination which would result in excision of the minivector sequence from the parent plasmid. An aliquot of the transformed strain is grown up at 30° C. in shaker flasks and used to inoculate a fermenter containing modified terrific broth medium.

Cells are grown at 30° C., maintaining the pH at 7 and the dissolved oxygen concentration above 60%, to ensure the cells remain in exponential phase. Once cells have reached mid-exponential phase, λ-Int expression is induced by shifting the culture to 43° C. for -30 minutes to induce λ-Int expression. The increased temperature leads to denaturation of the cI857 repressor, which prevents λ-Int expression at lower temperatures. λ-Int is not active at the higher 43° C. temperature, therefore the culture is subsequently shifted down to 30° C. to allow recombination to proceed for about an hour (1-4 hrs). Prior to the temperature shift back to the lower temperature, norfloxacin is added to the fermenter prevent decatenation of the recombination products by topoisomerase IV.

### MINIVECTOR PURIFICATION

**Step 1:** The first step in purification is to harvest the cells containing MiniVector by centrifugation.

**Step 2:** Cells are first incubated with lysozyme to break down the bacterial cell walls and then lysed using a standard alkaline lysis procedure.

**Step 3:** The nucleic acid (DNA and RNA) in the lysate is precipitated with isopropanol then resuspended to reduce the volume per usual procedures. Nucleic acid solution is then incubated with RNaseA to degrade the RNA, followed by incubation with proteinase K to degrade any residual proteins.

**Step 4:** Nucleic acid solution is incubated with polyethylene glycol (PEG) and NaCl and incubated on ice for ~15 minutes. By carefully controlling the concentration of PEG, larger DNA species are selectively precipitated while the smaller minivector DNA stays in solution. For the 339 bp MiniVector exemplified herein a solution containing an equal volume of 10% PEG-8000, 1.6 M NaCl was added to the nucleic acid solution (final concentrations: 5% PEG-8000, 0.8M NaCl). For larger MiniVectors lower concentrations of PEG are used. The precipitated larger DNA species is thus pelleted by centrifugation. The smaller nucleic acid (DNA and RNA) species in the supernatant are subsequently precipitated with ethanol to remove the PEG.

PEG precipitation is quick and has high capacity but has low resolution and can only separate DNA species significantly different in size (two-fold or more). It is used to remove a majority of the unwanted large circle (miniplasmid) recombination byproduct and any unrecombined parent plasmid. Reducing the mass of contaminating large DNA species makes subsequent downstream purification steps much more efficient.

**Step 5:** DNA is then further purified using anion-exchange chromatography, although other methods are available. We use Qiagen plasmid purification kits for this (e.g., Maxiprep kit or Gigaprep Kit) but columns from other manufacturers may be used. The major purpose of this step is to remove the (degraded) RNA and other (non-nucleic acid) contaminants, and it does not differentiate between different sized DNA species. Following anion-exchange the DNA is again precipitated with isopropanol and resuspended in a small volume for gel-filtration chromatography.

**Step 6: Gel-filtration.** This step completely removes any remaining large DNA contaminants, separating DNA according to size (larger DNA species eluting first). Although described in the original US7622252 patent, we have made several modifications since that patent was filed.

The contaminating DNA species are not able to enter the beads in the gel filtration matrix and are typically eluted in the "void volume," while the MiniVector DNA elutes later. Here, instead of using a single gel filtration column, two or three columns are connected in series such that when DNA is eluted from one column it enters the next column in the series. This significantly increases the separation of DNA species. Using multiple columns in series also allows different combinations of gel filtration resin to be used, thus optimizing size separation. For example, Sephacryl S-500 is best for separating MiniVector DNA from the parent plasmid. Sephacryl S-400 provides better separation of monomeric MiniVector from any multimeric length byproducts.

Using different columns in decreasing size separation range sequentially like this allows ultrapure monomeric MiniVector to be isolated and the recovery efficiency of DNA from gel-filtration is very high. Essentially all the DNA loaded onto the columns is eluted (provided that the DNA stays in solution). Therefore, there is no penalty in terms of yield for running the same DNA through the gel-filtration columns multiple times. To further remove any remaining contaminants, the DNA may simply be loaded again through the series of gel filtration columns.

Other purification methods can also be used in various combination(s), including high density centrifugation, dead end filtration, cross flow filtration, ultrafiltration, precipitations, binding to various resins, phenol-chloroform extraction; proteinase K digestion, electrophoresis, ion exchange chromatography, affinity chromatography, and the like, as well as techniques to be developed in the future.

### MINVECTOR PAYLOADS

Target sequences currently under development for gene therapy uses to treat liver cirrhosis and liver cancer include P53⁺, Relaxin⁺ FOXM1⁻, CAD11⁻, MDM2⁻, MDM4- and STATS-. Herein, the + sign means the protein/gene is upregulated in the therapy, usually by adding a copy of the gene under a strong promoter, or by upregulating the endogenous promoter, and the - sign meaning the protein/gene is downregulated, usually by adding an RNAi or by downregulating the endogenous promoter.

Additional targets that will be tested in the near future include transforming growth factor beta 1 (TGFB) (OMIM 190180) and signal transducer and activator of transcription 6 (STAT6) (OMIM 601512), which can be downregulated using an RNAi approach, possibly both in conjunction with other targets.

TGFB is a multifunctional peptide that controls proliferation, differentiation, and other functions in many cell types. TGFB acts synergistically with TGFA (OMIM 190170) in inducing transformation. It also acts as a negative autocrine growth factor. Dysregulation of TGFB activation and signaling may result in apoptosis. Many cells synthesize TGFB and almost all of them have specific receptors for this peptide. TGFB1, TGFB2 (190220), and TGFB3 (190230) all function through the same receptor signaling systems. TGFB is known to be important in wound healing and fibrosis is associated with increased expression of TGFB, making it a logical target, and the other members of the pathway may also prove useful.

Lipid metabolism, especially fatty acid oxidation (FAO) dysfunction, is a major driver of renal fibrosis; however, until recently the mechanisms remained unclear. Recently, scientists demonstrated an association between STAT6 and tubular lipid metabolism in fibrotic kidneys. Specifically, STAT6 was activated along with the accumulation of lipids via the downregulation of FAO-related genes when mice were subj ected to unilateral ureteral obstruction or high-fat diet challenge. Tubular-specific depletion, or pharmacologic inhibitor of STAT6 in mice, and STAT6 knockdown in cultured tubular cells attenuated lipid accumulation and renal fibrosis by enhancing FAO. Mechanistically, STAT6 transcriptionally inhibited the expression of PPARα (OMIM 170998) and its FAO-related target genes through a sis-inducible element located in the promoter region of the protein.

Although we focused initially on the above targets, there are many known liver specific targets that have known associations with liver disease, such as HGF (OMIM 604375), ATP7B (OMIM 606882), ABCB4 (OMIM 171060), ALDOB (OMIM 612724, GBE1 (OMIM 607839), FAH (OMIM 613871), ASL (OMIM 608310), SLC25A13 (OMIM 603859), LIPA (OMIM 613497), SERPINA1 (OMIM 107400), CFTR (602421), HFE (OMIM 613609), KRT18 (OMIM 215600), possibly MTDPS4A (OMIM 215600), CASP8 (OMIM 601763), CTNNB1 (OMIM 116806), PIK3CA(OMIM 171834), APC (OMIM 611731), IGF2R (OMIM 147280), MET (OMIM 164860), PDGFRL (OMIM 604584), AXIN (OMIM 603816), TP53 (OMIM 191170), FOCAD (OMIM 614606), MU (OMIM 613282), TRIM37 (OMIM 605073), MARS 1 (OMIM 156560), PBC1 (OMIM 109720), LBR (OMIM 600024), KIF12 (OMIM 611278), PBC3 (OMIM 613008), PCB4 (OMIM 614220), PCB5 (OMIM 614221), to name a few. There is insufficient time and space to describe each of these liver cirrhosis/cancer targets in detail, but each OMIM entry cited (and their links) are incorporated by reference in their entireties for all purposes. Further, the OMIM entries are hyperlinked to the relevant DNA and protein sequences, for use as targets in the MiniVectors described herein. Various inhibitory RNA sequences to these targets can be found in the many RNAi databases, such as RNACentral, GenomeRNAi.org, the RNAi Consortium's SHRNA library (stocks distributed by SigmaAldrich), the RNAiAtlas, DocleraWiki, NCBI, the RNAi Codex, to name just a few.

One payload for which we already have significant efficacy data is the FOXM1 shRNA (targeting 5'- ATAATTAGAGGATAATTTG-3' SEQ ID NO. 1). Additional shRNA payloads of strong interest are CDH11, and MDM2 and 4. Other payloads encode genes that promote apoptosis (e.g., p53).

Any shRNAs or other RNAi sequences can be designed using freely available, open access, algorithms (e.g., siRNA Wizard^{™} Software, siDESIGN Center, etc.) and then screened for off-target effects using NCBI-BLAST. Alternatively, there are significant numbers of commercially available sequences that can be used for initial proof of concept work. SigmaAldrich, for example has more than 200,000 individual sequences available, including more than 20,000 prescreened human specific sequences from the TRC1.5 and TRC2 collections.

Note that if the therapeutic sequence is shRNA, the promoter will likely be U6 or H1 or another promoter recognized by mammalian RNA polymerase III. If said therapeutic sequence is a gene (p53, p16, p21, p27, E2F genes, PTEN, caspase, or another apoptosis inducing gene), the promoter will be CMV, EF1α, or another promoter for mammalian RNA polymerase II. **Tables 1-6** show exemplary payload and MiniVector sequences. Additional sequences and various disease targets are discussed in US Serial No. 16/180,046, incorporated by reference in its entirety for all purposes.

| **Table 1.** Payload therapeutic sequences that may be encoded on an ultrapure MiniVector | | | | | |
|---|---|---|---|---|---|
| SEQ ID NO | | Gene | Description | Dharmacon Cat. No. | Mature Antisense |
| | 1. | FOXM1 | Forkhead box protein M1 Q08050 | V2LHS_283849 | ATAATTAGAGGATAATTTG |
| | 2. | FOXM1 | | V3LHS_396939 | ATTGTTGATAGTGCAGCCT |
| | 3. | FOXM1 | | V3LHS_396937 | TGAATCACAAGCATTTCCG |
| | 4. | FOXM1 | | V3LHS_396941 | TGATGGTCATGTTCCGGCG |
| | 5. | FOXM1 | | V3LHS_396940 | AATAATCTTGATCCCAGCT |
| | 6. | FOXM1 | | V3LHS_314369 | TACTGAGGAATATTGTGCT |
| | 7. | MDM4 | O15151 | V2LHS_11941 | TATGTACTGACCTAAATAG |
| | 8. | MDM4 | | V2LHS_151660 | ATCTGAATACCAATCCTTC |
| | 9. | MDM4 | | V3LHS_356802: | TGAACACTGAGCAGAGGTG |
| | 10. | MDM4 | | V3LHS_356797: | AACAGTGAACATTTCACCT |

**Table 2. MiniVector elements**

| **Table 2.** MiniVector elements | | | |
|---|---|---|---|
| Module | Element | Description | Use |
| A | *λ-att*L | *att*L from the λ-integrase system | Recombination sites (product of site-specific recombination used to generate MiniVector). Sequences listed in Table 3. |
| | λ-*att*R | *att*R from the λ-integrase system | |
| | λ-*att*B | *att*B from the λ-integrase system | |
| | λ-*att*P | *att*P from the λ-integrase system | |
| | *loxP* | *loxP* site for Cre recombinase | |
| | γδ-*res* | *res* site for the γδ (Tn1000) resolvase | |
| | FRT | FRT site for Flp recombinase | |
| | *hixL* | *hix*L site for Hin recombinase | |
| | *hixR* | *hixR* site for Hin recombinase | |
| | Tn3 *res* | *res* site for Tn3 resolvase | |
| | Tn21 *res* | *res* site for Tn21 resolvase | |
| | *cer* | *cer* site for XerCD system | |
| | *psi* | *psi* site for XerCD | |
| B | | Tissue-specific promoter of alcohol dehydrogenase 1 (ALDH1) | Initiation of transcription. Includes promoters for RNA polymerase II and RNA polymerase III. Full sequences of selected promoters provided in Table 4. |
| | AMY1C | Tissue-specific promoter of human amylase alpha 1C (AMY1C) | |
| | β-actin | Promoter from the (human) beta actin gene | |
| | CaMKIIα | Ca2+/calmodulin-dependent protein kinase II alpha promoter | |
| | CMV | Promoter from the human cytomegalovirus (CMV) | |
| | Mini CMV | Minimized version of CMV | |
| | CAG | CMV early enhancer/chicken β actin promoter (CAG). Synthetic hybrid promoter made from a) the CMV early | |
| | | enhancer element, b) the promoter, the first exon and the first intron of chicken beta-actin gene, and c) the splice acceptor of the rabbit beta-globin gene | |
| | Cyto-keratin 18 and 19 | Cell-specific promoters of the human keratin 18 and 19 genes | |
| | EF1α | Strong expression promoter from human elongation factor 1 alpha | |
| | GFAP | Tissue-specific promoter of the glial fibrillary acidic protein (GFAP) | |
| | | Promoter from the human polymerase III RNA promoter | |
| | Kallikrein | Tissue-specific promoter of the kallikrein gene. | |
| | NFK-β | Nuclear factor kappa-light-chain-enhancer of activated B cells (NF-Kβ) | |
| | PGK1 | Promoter from human or mouse phosphoglycerate kinase gene (PGK) | |
| | RSV | Long terminal repeat (LTR) of the rous sarcoma virus (RSV) | |
| | SV40 | Mammalian expression promoter from the simian vacuolating virus 40 | |
| | UBC | Promoter of the human ubiquitin C gene (UBC) | |
| | U6 | Promoter from the human U6 small nuclear promoter | |
| C | shRNA | (DNA) sequence encoding short hairpin RNA (shRNA) transcript. Sequences for use in target validation are listed in Table 1. Potential therapeutic sequences will be designed *de novo* and optimized for knockdown efficiency. | Knockdown of gene expression through RNA interference |
| | miRNA | (DNA) sequence encoding micro-RNA (miRNA) transcript | |
| | IhRNA | (DNA) sequence encoding long hairpin RNA (IhRNA) transcript | |
| | IncRNA | (DNA) sequence encoding long non-coding RNA (lncRNA) transcript | Knockdown of gene expression (not RNAi) |
| | piRNA | (DNA) sequence encoding piwi-interacting (piRNA) RNA transcript | |
| D | | Transcriptional terminator sequence (Any can be used) | |
| E | S/MAR | Scaffold/matrix attached region from eukaryotic chromosomes (Sequences in Table 5) | Episomal replication |
| | CpG motifs | Unmethylated deoxycytidyl-deoxyguanosine (CpG) dinucleotides: (Sequences in Table 5) | Immunostimulatory activity |
| F/G | β-globin intron | Intron of the human β globin gene (130 bp) | Gene expression enhancer |
| | HGH intron | Intron of the human growth hormone gene (262 bp) | |
| H | SV40 early promoter | Simian virus 40 early promoter (351 bp) | Nuclear localization |
| | NF-κβ | Binding site of nuclear factor kappa-light-chain-enhancer of activated B cells (55 bp (5 repeats of | |
| | | GGGGACTTTCC SEQ ID NO 11)) | |
| | p53 NLS | Binding site of tumor protein 53 (p53): AGACTGGGCATGTCTGGGCA SEQ ID NO 12 | |
| | p53 NLS | Binding site of tumor protein 53 (p53): GAACATGTCCCAACATGTTG SEQ ID NO 13 | |
| | Adenovirus major late promoter | GGGGCTATAAAAGGG SEQ ID NO 14 | |

**Table 3. Complete sequences for element A (underline = recombination sites)**

| SEQ ID NO | Site | Sequence (5'-3') |
|---|---|---|
| 15. | λ*-att*L | TCCGTTGAAGCCTGCTTTTTTATACTAAGTTGGCATTATAAAAAAGCATTG CTTATCAATTTGTTGCAACGAACAGGTCACTATCAGTCAAAATAAAATCAT TATT |
| 16. | λ*-att*R | AGATGCCTCAGCTCTGTTACAGGTCACTAATACCATCTAAGTAGTTGATTC ATAGTGACTGCATATGTTGTGTTTTACAGTATTATGTAGTCTGTTTTTTATG CAAAATCTAATTTAATATATTGATATTTATATCATTTTACGTTTCTCGTTCAG CTTTTTTATACTAACTTGAGCGAAACG |
| 17. | λ*-att*B | TCCGTTGAAGCCTGCTTTTTTATACTAACTTGAGCGAAACG |
| 18. | λ*-att*P | AGATGCCTCAGCTCTGTTACAGGTCACTAATACCATCTAAGTAGTTGATTC ATAGTGACTGCATATGTTGTGTTTTACAGTATTATGTAGTCTGTTTTTTATG CAAAATCTAATTTAATATATTGATATTTATATCATTTTACGTTTCTCGTTCAG CTTTTTTATACTAAGTTGGCATTATAAAAAAGCATTGCTTATCAATTTGTTG CAACGAACAGGTCACTATCAGTCAAAATAAAATCATTATT |
| 19. | *loxP* | ATAACTTCGTATAGCATACATTATACGAAGTTAT |
| 20. | γδ-*res* | ATTTTGCAACCGTCCGAAATATTATAAATTATCGCACACATAAAAACAGTG CTGTTAATGTGTCTATTAAATCGATTTTTTGTTATAACAGACACTGCTTGTC CGATATTTGATTTAGGATACATTTTTA |
| 21. | FRT | GAAGTTCCTATTCTCTAGAAAGTATAGGAACTTC |
| 22. | *hixL* | TTCTTGAAAACCAAGGTTTTTGATAA |
| 23. | *hixR* | TTTTCCTTTTGGAAGGTTTTTGATAA |
| 24. | Tn3 *res* | CAACCGTTCGAAATATTATAAATTATCAGACATAGTAAAACGGCTTCGTTT GAGTGTCCATTAAATCGTCATTTTGGCATAATAGACACATCGTGTCTGATA TTCGATTTAAGGTACATTT |
| 25. | Tn21 *res* | GCCGCCGTCAGGTTGAGGCATACCCTAACCTGATGTCAGATGCCATGTG TAAATTGCGTCAGGATAGGATTGAATTTTGAATTTATTGACATATCTCGTT GAAGGTCATAGAGTCTTCCCTGACAT |
| 26. | | GGTGCGTACAATTAAGGGATTATGGTAAAT |
| 27. | | GGTGCGCGCAAGATCCATTATGTTAAAC |

**Table 4. Complete sequences for element B (promoters)**

| SEQ ID NO | Promoter | Sequence (5'-3') |
|---|---|---|
| 28. | CMV | GACATTGATTATTGACTAGTTATTAATAGTAATCAATTACGGGGTCATTAG TTCATAGCCCATATATGGAGTTCCGCGTTACATAACTTACGGTAAATGGC CCGCCTGGCTGACCGCCCAACGACCCCCGCCCATTGACGTCAATAATGA CGTATGTTCCCATAGTAACGCCAATAGGGACTTTCCATTGACGTCAATGG GTGGAGTATTTACGGTAAACTGCCCACTTGGCAGTACATCAAGTGTATCA TATGCCAAGTACGCCCCCTATTGACGTCAATGACGGTAAATGGCCCGCC TGGCATTATGCCCAGTACATGACCTTATGGGACTTTCCTACTTGGCAGTA CATCTACGTATTAGTCATCGCTATTACCATGGTGATGCGGTTTTGGCAGT ACATCAATGGGCGTGGATAGCGGTTTGACTCACGGGGATTTCCAAGTCT CCACCCCATTGACGTCAATGGGAGTTTGTTTTGGCACCAAAATCAACGG GACTTTCCAAAATGTCGTAACAACTCCGCCCCATTGACGCAAATGGGCG GTAGGCGTGTACGGTGGGAGGTCTATATAAGCAGAGCT |
| 29. | mini-CMV | CCAAAATCAACGGGACTTTCCAAAATGTCGTAACAACTCCGCCCCATTGA CGCAAATGGGCGGTAGGCGTGTACGGTGGGAGGTCTATATAAGCAGAG CT |
| 30. | RSV | GGTGCACACCAATGTGGTGAATGGTCAAATGGCGTTTATTGTATCGAGC TAGGCACTTAAATACAATATCTCTGCAATGCGGAATTCAGTGGTTCGTCC AATCCATGTCAGACCCGTCTGTTGCCTTCCTAATAAGGCACGATCGTACC ACCTTACTTCCACCAATCGGCATGCACGGTGCTTTTTCTCTCCTTGTAAG GCATGTTGCTAACTCATCGTTACCATGTTGCAAGACTACAAGAGTATTGC ATAAGACTACATT |
| 31. | CAG | GCGTTACATAACTTACGGTAAATGGCCCGCCTGGCTGACCGCCCAACGA CCCCCGCCCATTGACGTCAATAATGACGTATGTTCCCATAGTAACGCCAA TAGGGACTTTCCATTGACGTCAATGGGTGGAGTATTTACGGTAAACTGCC CACTTGGCAGTACATCAAGTGTATCATATGCCAAGTACGCCCCCTATTGA CGTCAATGACGGTAAATGGCCCGCCTGGCATTATGCCCAGTACATGACC TTATGGGACTTTCCTACTTGGCAGTACATCTACGTATTAGTCATCGCTATT ACCATGGTCGAGGTGAGCCCCACGTTCTGCTTCACTCTCCCCATCTCCC CCCCCTCCCCACCCCCAATTTTGTATTTATTTATTTTTTAATTATTTTGTGC AGCGATGGGGGCGGGGGGGGGGGGGGGGCGCGCGCCAGGCGGGGC GGGGCGGGGCGAGGGGCGGGGCGGGGCGAGGCGGAGAGGTGCGGC GGCAGCCAATCAGAGCGGCGCGCTCCGAAAGTTTCCTTTTATGGCGAG GCGGCGGCGGCGGCGGCCCTATAAAAAGCGAAGCGCGCGGCGGGCG |
| 32. | EF1a | GCTCCGGTGCCCGTCAGTGGGCAGAGCGCACATCGCCCACAGTCCCCG AGAAGTTGGGGGGAGGGGTCGGCAATTGAACCGGTGCCTAGAGAAGGT GGCGCGGGGTAAACTGGGAAAGTGATGTCGTGTACTGGCTCCGCCTTTT TCCCGAGGGTGGGGGAGAACCGTATATAAGTGCAGTAGTCGCCGTGAA CGTTCTTTTTCGCAACGGGTTTGCCGCCAGAACACAGGTAAGTGCCGTG TGTGGTTCCCGCGGGCCTGGCCTCTTTACGGGTTATGGCCCTTGCGTGC CTTGAATTACTTCCACGCCCCTGGCTGCAGTACGTGATTCTTGATCCCGA GCTTCGGGTTGGAAGTGGGTGGGAGAGTTCGAGGCCTTGCGCTTAAGG AGCCCCTTCGCCTCGTGCTTGAGTTGAGGCCTGGCCTGGGCGCTGGGG CCGCCGCGTGCGAATCTGGTGGCACCTTCGCGCCTGTCTCGCTGCTTTC GATAAGTCTCTAGCCATTTAAAATTTTTGATGACCTGCTGCGACGCTTTTT TTCTGGCAAGATAGTCTTGTAAATGCGGGCCAAGATCTGCACACTGGTAT TTCGGTTTTTGGGGCCGCGGGCGGCGACGGGGCCCGTGCGTCCCAGC GCACATGTTCGGCGAGGCGGGGCCTGCGAGCGCGGCCACCGAGAATC GGACGGGGGTAGTCTCAAGCTGGCCGGCCTGCTCTGGTGCCTGGCCTC GCGCCGCCGTGTATCGCCCCGCCCTGGGCGGCAAGGCTGGCCCGGTC GGCACCAGTTGCGTGAGCGGAAAGATGGCCGCTTCCCGGCCCTGCTGC AGGGAGCTCAAAATGGAGGACGCGGCGCTCGGGAGAGCGGGCGGGTG AGTCACCCACACAAAGGAAAAGGGCCTTTCCGTCCTCAGCCGTCGCTTC |
| | | ATGTGACTCCACGGAGTACCGGGCGCCGTCCAGGCACCTCGATTAGTTC TCGAGCTTTTGGAGTACGTCGTCTTTAGGTTGGGGGGAGGGGTTTTATG CGATGGAGTTTCCCCACACTGAGTGGGTGGAGACTGAAGTTAGGCCAG CTTGGCACTTGATGTAATTCTCCTTGGAATTTGCCCTTTTTGAGTTTGGAT CTTGGTTCATTCTCAAGCCTCAGACAGTGGTTCAAAGTTTTTTTCTTCCAT TTCAGGTGTCGTGA |
| 33. | EFS | ATCGATTGGCTCCGGTGCCCGTCAGTGGGCAGAGCGCACATCGCCCAC AGTCCCCGAGAAGTTGGGGGGAGGGGTCGGCAATTGAACCGGTGCCTA GAGAAGGTGGCGCGGGGTAAACTGGGAAAGTGATGTCGTGTACTGGCT CCGCCTTTTTCCCGAGGGTGGGGGAGAACCGTATATAAGTGCAGTAGTC GCCGTGAACGTTCTTTTTCGCAACGGGTTTGCCGCCAGAACACAGGTGT CGTGACGCG |
| 34. | Human β-actin | GGCCTCCGCGCCGGGTTTTGGCGCCTCCCGCGGGCGCCCCCCTCCTCA CGGCGAGCGCTGCCACGTCAGACGAAGGGCGCAGCGAGCGTCCTGAT CCTTCCGCCCGGACGCTCAGGACAGCGGCCCGCTGCTCATAAGACTCG GCCTTAGAACCCCAGTATCAGCAGAAGGACATTTTAGGACGGGACTTGG GTGACTCTAGGGCACTGGTTTTCTTTCCAGAGAGCGGAACAGGCGAGGA AAAGTAGTCCCTTCTCGGCGATTCTGCGGAGGGATCTCCGTGGGGCGG TGAACGCCGATGATTATATAAGGACGCGCCGGGTGTGGCACAGCTAGTT CCGTCGCAGCCGGGATTTGGGTCGCGGTTCTTGTTTGTGGATCGCTGTG ATCGTCACTTGGTGAGTAGCGGGCTGCTGGGCTGGCCGGGGCTTTCGT GGCCGCCGGGCCGCTCGGTGGGACGGAAGCGTGTGGAGAGACCGCCA AGGGCTGTAGTCTGGGTCCGCGAGCAAGGTTGCCCTGAACTGGGGGTT GGGGGGAGCGCAGCAAAATGGCGGCTGTTCCCGAGTCTTGAATGGAAG ACGCTTGTGAGGCGGGCTGTGAGGTCGTTGAAACAAGGTGGGGGGCAT GGTGGGCGGCAAGAACCCAAGGTCTTGAGGCCTTCGCTAATGCGGGAA AGCTCTTATTCGGGTGAGATGGGCTGGGGCACCATCTGGGGACCCTGA CGTGAAGTTTGTCACTGACTGGAGAACTCGGTTTGTCGTCTGTTGCGGG GGCGGCAGTTATGGCGGTGCCGTTGGGCAGTGCACCCGTACCTTTGGG AGCGCGCGCCCTCGTCGTGTCGTGACGTCACCCGTTCTGTTGGCTTATA ATGCAGGGTGGGGCCACCTGCCGGTAGGTGTGCGGTAGGCTTTTCTCC GTCGCAGGACGCAGGGTTCGGGCCTAGGGTAGGCTCTCCTGAATCGAC AGGCGCCGGACCTCTGGTGAGGGGAGGGATAAGTGAGGCGTCAGTTTC TTTGGTCGGTTTTATGTACCTATCTTCTTAAGTAGCTGAAGCTCCGGTTTT GAACTATGCGCTCGGGGTTGGCGAGTGTGTTTTGTGAAGTTTTTTAGGC ACCTTTTGAAATGTAATCATTTGGGTCAATATGTAATTTTCAGTGTTAGAC TAGTAAATTGTCCGCTAAATTCTGGCCGTTTTTGGCTTTTTTGTTAGAC |
| 35. | NFK-β | GCTAGCGGGAATTTCCGGGAATTTCCGGGAATTTCCGGGAATTTCCAGA TCTGCCGCCCCGACTGCATCTGCGTGTTCGAATTCGCCAATGACAAGAC GCTGGGCGGGGTTTGTGTCATCATAGAACTAAAGACATGCAAATATATTT CTTCCGGGGACACCGCCAGCAAACGCGAGCAACGGGCCACGGGGATG AAGCAGAAGCTTGGCA |
| 36. | Ubiquitin-C | GTCTAACAAAAAAGCCAAAAACGGCCAGAATTTAGCGGACAATTTACTAG TCTAACACTGAAAATTACATATTGACCCAAATGATTACATTTCAAAAGGTG CCTAAAAAACTTCACAAAACACACTCGCCAACCCCGAGCGCATAGTTCAA AACCGGAGCTTCAGCTACTTAAGAAGATAGGTACATAAAACCGACCAAA GAAACTGACGCCTCACTTATCCCTCCCCTCACCAGAGGTCCGGCGCCTG TCGATTCAGGAGAGCCTACCCTAGGCCCGAACCCTGCGTCCTGCGACG GAGAAAAGCCTACCGCACACCTACCGGCAGGTGGCCCCACCCTGCATT ATAAGCCAACAGAACGGGTGACGTCACGACACGACGAGGGCGCGCGCT CCCAAAGGTACGGGTGCACTGCCCAACGGCACCGCCATAACTGCCGCC CCCGCAACAGACGACAAACCGAGTTCTCCAGTCAGTGACAAACTTCACG TCAGGGTCCCCAGATGGTGCCCCAGCCCATCTCACCCGAATAAGAGCTT TCCCGCATTAGCGAAGGCCTCAAGACCTTGGGTTCTTGCCGCCCACCAT |
| | | GCCCCCCACCTTGTTTCAACGACCTCACAGCCCGCCTCACAAGCGTCTT CCATTCAAGACTCGGGAACAGCCGCCATTTTGCTGCGCTCCCCCCAACC CCCAGTTCAGGGCAACCTTGCTCGCGGACCCAGACTACAGCCCTTGGC GGTCTCTCCACACGCTTCCGTCCCACCGAGCGGCCCGGCGGCCACGAA AGCCCCGGCCAGCCCAGCAGCCCGCTACTCACCAAGTGACGATCACAG CGATCCACAAACAAGAACCGCGACCCAAATCCCGGCTGCGACGGAACTA GCTGTGCCACACCCGGCGCGTCCTTATATAATCATCGGCGTTCACCGCC CCACGGAGATCCCTCCGCAGAATCGCCGAGAAGGGACTACTTTTCCTCG CCTGTTCCGCTCTCTGGAAAGAAAACCAGTGCCCTAGAGTCACCCAAGT CCCGTCCTAAAATGTCCTTCTGCTGATACTGGGGTTCTAAGGCCGAGTC TTATGAGCAGCGGGCCGCTGTCCTGAGCGTCCGGGCGGAAGGATCAGG ACGCTCGCTGCGCCCTTCGTCTGACGTGGCAGCGCTCGCCGTGAGGAG GGGGGCGCCCGCGGGAGGCGCCAAAACCCGGCGCGGAGGC |
| 37. | SV40 | GGTGTGGAAAGTCCCCAGGCTCCCCAGCAGGCAGAAGTATGCAAAGCA TGCATCTCAATTAGTCAGCAACCAGGTGTGGAAAGTCCCCAGGCTCCCC AGCAGGCAGAAGTATGCAAAGCATGCATCTCAATTAGTCAGCAACCATA GTCCCGCCCCTAACTCCGCCCATCCCGCCCCTAACTCCGCCCAGTTCCG CCCATTCTCCGCCCCATGGCTGACTAATTTTTTTTATTTATGCAGAGGCC GAGGCCGCCTCGGCCTCTGAGCTATTCCAGAAGTAGTGAGGAGGCTTTT TTGGAGGCCTAGGCTTTTGCAAA |
| 38. | PGK | CCGGTAGGCGCCAACCGGCTCCGTTCTTTGGTGGCCCCTTCGCGCCAC CTTCTACTCCTCCCCTAGTCAGGAAGTTCCCCCCCGCCCCGCAGCTCGC GTCGTGCAGGACGTGACAAATGGAAGTAGCACGTCTCACTAGTCTCGTG CAGATGGACAGCACCGCTGAGCAATGGAAGCGGGTAGGCCTTTGGGGC AGCGGCCAATAGCAGCTTTGCTCCTTCGCTTTCTGGGCTCAGAGGCTGG GAAGGGGTGGGTCCGGGGGCGGGCTCAGGGGCGGGCTCAGGGGCGG GGCGGGCGCCCGAAGGTCCTCCGGAGGCCCGGCATTCTGCACGCTTCA AAAGCGCACGTCTGCCGCGCTGTTCTCCTCTTCCTCATCTCCGGGCCTT TCGACCTGCAGCC |
| 39. | H1 | AATATTTGCATGTCGCTATGTGTTCTGGGAAATCACCATAAACGTGAAAT GTCTTTGGATTTGGGAATCTTATAAGTTCTGTATGAGACCACAGATCCC |
| 40. | U6 | GATCCGACGCCGCCATCTCTAGGCCCGCGCCGGCCCCCTCGCACAGAC TTGTGGGAGAAGCTCGGCTACTCCCCTGCCCCGGTTAATTTGCATATAAT ATTTCCTAGTAACTATAGAGGCTTAATGTGCGATAAAAGACAGATAATCT GTTCTTTTTAATACTAGCTACATTTTACATGATAGGCTTGGATTTCTATAA GAGATACAAATACTAAATTATTATTTTAAAAAACAGCACAAAAGGAAACTC ACCCTAACTGTAAAGTAATTGTGTGTTTTGAGACTATAAATATCCCTTGGA GAAAAGCCTTGTT |

**Table 5. Complete sequences for elements E, F and G (accessory sequences)**

| SEQ ID NO | Element | Sequence (5'-3') |
|---|---|---|
| 41. | 250 bp S/MAR | TCTTTAATTTCTAATATATTTAGAATCTTTAATTTCTAATATATTTAGA ATCTTTAATTTCTAATATATTTAGAATCTTTAATTTCTAATATATTTAG AATCTTTAATTTCTAATATATTTAGAATCTTTAATTTCTAATATATTTA GAATCTTTAATTTCTAATATATTTAGAATCTTTAATTTCTAATATATTT AGAATCTTTAATTTCTAATATATTTAGAATCTTTAATTTCTAATATATT TAGAA |
| 42. | 439 bp S/MAR | TCTTTAATTTCTAATATATTTAGAATCTTTAATTTCTAATATATTTAGA ATCTTTAATTTCTAATATATTTAGAATCTTTAATTTCTAATATATTTAG AATCTTTAATTTCTAATATATTTAGAATCTTTAATTTCTAATATATTTA GAATCTTTAATTTCTAATATATTTAGAATCTTTAATTTCTAATATATTT AGAATCTTTAATTTCTAATATATTTAGAATCTTTAATTTCTAATATATT TAGAA |
| 43. | (45 bp) Type A | |
| 44. | Cpg motif (24 bp) Type B Cpg motif | TCGTCGTTTTGTCGTTTTGTCGTT |
| 45. | (21 bp) Type C CpG motif | TCGTCGAACGTTCGAGATGAT |
| 46. | β-globin intron | GTTGGTATCAAGGTTACAAGACAGGTTTAAGGAGACCAATAGAAAC TGGGCATGTGGAGACAGAGAAGACTCTTGGGTTTCTGATAGGCAC TGACTCTCTCTGCCTATTGGTCTATTTTCCCACCCTTAG |
| 47. | Human growth hormone intron | TTCGAACAGGTAAGCGCCCCTAAAATCCCTTTGGGCACAATGTGT CCTGAGGGGAGAGGCAGCGACCTGTAGATGGGACGGGGGCACTA ACCCTCAGGTTTGGGGCTTCTGAATGTGAGTATCGCCATGTAAGC CCAGTATTTGGCCAATCTCAGAAAGCTCCTGGTCCCTGGAGGGAT GGAGAGAGAAAAACAAACAGCTCCTGGAGCAGGGAGAGTGCTGG CCTCTTGCTCTCCGGCTCCCTCTGTTGCCCTCTGGTTTC |

| Table 6: Relaxin and P53 sequences | | |
|---|---|---|
| | SEQ ID No | cDNA Sequence (5' to 3') |
| RLN2 (human) | 48. | ATGCCTCGCCTGTTTTTTTTCCACCTGCTAGGAGTCTGTTTACTACTGAA CCAATTTTCCAGAGCAGTCGCGGACTCATGGATGGAGGAAGTTATTAAA TTATGCGGCCGCGAATTAGTTCGCGCGCAGATTGCCATTTGCGGCATG AGCACCTGGAGCAAAAGGTCTCTGAGCCAGGAAGATGCTCCTCAGACA CCTAGACCAGTGGCAGAAATTGTGCCATCCTTCATCAACAAAGATACAG AAACCATAAATATGATGTCAGAATTTGTTGCTAATTTGCCACAGGAGCT GAAGTTAACCCTGTCTGAGATGCAGCCAGCATTACCACAGCTACAACAA CATGTACCTGTATTAAAAGATTCCAGTCTTCTCTTTGAAGAATTTAAGAA ACTTATTCGCAATAGACAAAGTGAAGCCGCAGACAGCAGTCCTTCAGAA TTAAAATACTTAGGCTTGGATACTCATTCTCGAAAAAAGAGACAACTCTA CAGTGCATTGGCTAATAAATGTTGCCATGTTGGTTGTACCAAAAGATCT CTTGCTAGATTTTGCTGA |
| RLN1 (mouse) | 49. | ATGTCCAGCAGATTTTTGCTCCAGCTCCTGGGGTTCTGGCTATTGCTGA GCCAGCCTTGCAGGACGCGAGTCTCGGAGGAGTGGATGGACGGATTC ATTCGGATGTGCGGCCGTGAATATGCCCGTGAATTGATCAAAATCTGC GGGGCCTCCGTGGGAAGATTGGCTTTGAGCCAGGAGGAGCCAGCTCT GCTTGCCAGGCAAGCCACTGAAGTTGTGCCATCCTTCATCAACAAAGAT GCAGAGCCTTTCGATACGACGCTGAAATGCCTTCCAAATTTGTCTGAAG AGCTCAAGGCAGTACTGTCTGAGGCTCAGGCCTCGCTCCCAGAGCTAC AACACGCACCTGTGTTGAGCGATTCTGTTGTTAGCTTGGAAGGCTTTAA GAAAACTCTCCATGATAAACTGGGTGAAGCAGAAGACGGCAGTCCTCC AGGGCTTAAATACTTGCAATCAGATACCCATTCACGGAAAAAGAGGGAG TCTGGTGGATTGATGAGCCAGCAATGTTGCCACGTCGGTTGTAGCAGA AGATCTATTGCTAAACTCTATTGC |
| | SEQ ID No | Amino Acid Sequence (5' to 3') |
| P53 isoform a | 50. | |
| | 51. | MFCQLAKTCP VQLWVDSTPP PGTRVRAMAI YKQSQHMTEV VRRCPHHERC SDSDGLAPPQ HLIRVEGNLR VEYLDDRNTF RHSVVVPYEP PEVGSDCTTI HYNYMCNSSC MGGMNRRPIL TIITLEDSSG NLLGRNSFEV RVCACPGRDR RTEEENLRKK GEPHHELPPG STKRALPNNT SSSPQPKKKP LDGEYFTLQI RGRERFEMFR ELNEALELKD AQAGKEPGGS RAHSSHLKSK KGQSTSRHKK LMFKTEGPDS D |

| Table 7: Additional expression sequences for STAT6 and TGFB1 targets | | |
|---|---|---|
| Gene | SEQ ID NO | The bold underlined TTCAAGAGA is the 9-mer loop of the stemloop of the shRNA structure. This sequence is from pSUPER. |
| Mouse STAT6 | 52. | AGACCTGTCCATTCGCTCA**TTCAAGAGA**TGAGCGAATGGACAGGTCT |
| Mouse STAT6 | 53. | CTGATGTGCGGCAAGATGC**TTCAAGAGA**GCATCTTGCCGCACATCAG |
| Mouse STAT6 | 54. | AGCCGGGTCTCCCTTACTC**TTCAAGAGA**GAGTAAGGGAGACCCGGCT |
| Mouse STAT6 | 55. | GTTGCATGGAAGCATCAGG**TTCAAGAGA**CCTGATGCTTCCATGCAAC |
| Human TGFB1 | 56. | CAATTCCTGGCGATACCTC**TTCAAGAGA**GAGGTATCGCCAGGAATTG |
| Human TGFB1 | 57. | GAGCGCACGATCATGTTGG**TTCAAGAGA**CCAACATGATCGTGCGCTC |
| Human TGFB1 | 58. | TGCCGCACGCAGCAGTTCT**TTCAAGAGA**AGAACTGCTGCGTGCGGCA |
| Human TGFB1 | 59. | AAGATAACCACTCTGGCGA**TTCAAGAGA**TCGCCAGAGTGGTTATCTT |
| Mouse TGFB1 | 60. | TCCCGAATGTCTGACGTAT**TTCAAGAGA**ATACGTCAGACATTCGGGA |
| Mouse TGFB1 | 61. | AAGACAGCCACTCAGGCGT**TTCAAGAGA**ACGCCTGAGTGGCTGTCTT |
| Mouse TGFB1 | 62. | TTCGATGCGCTTCCGTTTC**TTCAAGAGA**GAAACGGAAGCGCATCGAA |
| Mouse TGFB1 | 63. | TTGGCATGGTAGCCCTTGG**TTCAAGAGA**CCAAGGGCTACCATGCCAA |

The following references are incorporated by reference in their entirety for all purposes.
Catanese, D.J., et al., Supercoiled MiniVector DNA resists shear forces associated with gene therapy delivery, Gene Ther. 19(1): 94-100 (2012).
Darquet A.M., et al., Minicircle: an improved DNA molecule for in vitro and in vivo gene transfer, Gene Ther., 6: 209-218 (1999).
Fogg, J.M., et al., Exploring writhe in supercoiled minicircle DNA. J. Phys.-Condes. Matter, 18: S145-S159 (2006).
Hardee, C.L., Advances in non-viral DNA vectors for gene therapy, Genes 8, 65 (2017)
Hornstein, B.D., et al., Effects of circular DNA length on transfection efficiency by electroporation into HeLa cells, PLoS One. 11(12): e0167537 (2016).
Lis and Schleif, Size fractionation of double-stranded DNA by precipitation with polyethylene glycol. Nucleic Acids Research. 2, 383-389 (1975).
US20150376645, US20140056868, 61/653,279, filed May 30, 2012, Supercoiled MiniVectors as a tool for DNA repair, alteration and replacement
US8460924, US8729044, US9267150, US20110160284, US20120302625, US20130316449, 61/252,455, filed Oct. 16, 2009, Supercoiled MiniVectors for gene therapy applications
US7622252, US20070020659, 60/689,298, filed Jun. 10, 2005, Generation of minicircle DNA with physiological supercoiling
63/243,087 Ultrapure minivectors for gene therapy
US20060211117 Methods of making minicircles
WO1994009127 Supercoiled minicircle DNA as a unitary promoter vector
WO2002083889 Methods for the production of minicircles
Ramamoorth M., & Narvekar, A., Non viral vectors in gene therapy- An overview, J. Clinical & Diagnostic Res. 2015 Jan, Vol-9(1): GE01-GE06.
Hidai C., & Kitano, H., Nonviral gene therapy for cancer: A review, Diseases 2018, 6, 57.

The following UniProt cites include all sequences taught/linked therein:
P04637, P04090, Q08050, P55287, Q00987, 015151, P40763.

## Claims

1. A composition comprising a MiniVector plus a pharmaceutically acceptable carrier, said MiniVector being a double-stranded, supercoiled circular DNA lacking a bacterial origin of replication or an antibiotic selection gene, having a length of about 250-600 base pairs exclusive of expressible payload and having < 1% contamination by a parent plasmid DNA, said expressible payload being a sequence selected from one or more that upregulates one or more of P53 or relaxin, or downregulates one or more of FOXM1, CAD11, MDM2, MDM4, or STATS.

2. The ultrapure MiniVector of claim 1, wherein contamination by parent DNA is <0.1%.

3. The ultrapure MiniVector of claim 1, wherein contamination by parent DNA is <0.02% and is assessed by gel electrophoresis and staining at a sensitivity of ≤ 0.1 ng, or preferably ≤ 0.01 ng.

4. The ultrapure MiniVector of claim 1, wherein contamination by parent DNA is <0.02% and is assessed by gel electrophoresis and staining with SYBR Gold staining at a sensitivity of ≤ 0.1 ng.

5. The ultrapure MiniVector of claim 1, wherein said MiniVector is separated from said parent plasmid and recombination side-products on the basis of size, and does not use sequence-specific endonuclease cleavage *in vivo* for preparation of said MiniVector.

6. The ultrapure MiniVector of claim 1, wherein contamination by parent DNA is <0.02% and wherein said MiniVector is separated from said parent plasmid and recombination side-products on the basis of size, and does not use sequence-specific endonuclease cleavage *in vivo* for preparation of said MiniVector.

7. The ultrapure MiniVector of claim 1, wherein said MiniVector is purified by cross flow filtration or by PEG precipitation of large DNA or by at least two passes through multiple gel-filtration columns using progressively smaller size range size exclusion resins or by a combination thereof.

8. The ultrapure MiniVector of claim 1, wherein said MiniVector is purified by PEG precipitation of larger DNA species followed by anion exchange chromatography to remove RNA and non-nucleic acid components, followed by at least two passes through multiple gel-filtration columns using progressively smaller size range size exclusion resins.

9. The ultrapure MiniVector of claim 1, wherein said MiniVector is purified by PEG precipitation, anion exchange chromatography, and at least two passes through multiple gel-filtration columns using progressively smaller size range size exclusion resins, and one or more alcohol precipitations.

10. The ultrapure MiniVector of claim 1, comprising a promoter operably connected to said payload operably connected to a terminator.

11. The MiniVector of claim 1, wherein said MiniVector is ≤500 bp in length, excluding said payload.

12. A composition of claim 1 for use in a method of treating liver fibrosis or liver cancer, wherein the method comprises administering the composition to a patient having liver fibrosis or liver cancer in an amount sufficient to upregulate one or more of P53 or relaxin by at least 10% as assessed by protein activity, or in an amount sufficient to downregulate one or more of FOXM1, CAD11, MDM2, MDM4, or STATS by at least 10% as assessed by protein activity.

13. The composition for the use of claim 12, wherein said administration is by injection into or onto a liver of said patient; or
wherein said administration is by surgical entry into a liver of said patient; or
wherein said administration is by IV delivery, lavage of a liver, or surface coating of the liver; or
wherein said administration occurs a plurality of times; or
wherein said administration occurs a plurality of times 1-4 weeks apart.

14. A composition comprising a MiniVector plus a pharmaceutically acceptable carrier, said MiniVector being a double-stranded, supercoiled circular DNA lacking a bacterial origin of replication or an antibiotic selection gene, having a length of about 250-600 base pairs exclusive of expressible payload and having < 1% contamination by a parent plasmid DNA, said expressible payload being a sequence selected from one or more that upregulates one or more of P53 or relaxin, or downregulates one or more of FOXM1, CAD11, MDM2, MDM4, STAT3, STAT6 or TGFB1.

15. The composition of claim 14 for use in a method of treating liver fibrosis or liver cancer, wherein the method comprises administering the composition of any of claim 18 to a patient having liver fibrosis or liver cancer in an amount sufficient to upregulate one or more of P53 or relaxin by at least 10% as assessed by protein activity, or in an amount sufficient to downregulate one or more of FOXM1, CAD11, MDM2, MDM4, STAT3, STAT6 or TGFB1 by at least 10% as assessed by protein activity.
